(19) Europäisches Patentamt
European Patent Office
Office européen des brevets

(11) Veröffentlichungsnummer: **0 658 628 A1**

# (12) EUROPÄISCHE PATENTANMELDUNG

(21) Anmeldenummer: **94118848.4**

(22) Anmeldetag: **30.11.94**

(51) Int. Cl.[6]: **C12P 7/56**, C07C 59/08

(30) Priorität: **08.12.93 DE 4341770**

(43) Veröffentlichungstag der Anmeldung:
**21.06.95 Patentblatt 95/25**

(84) Benannte Vertragsstaaten:
**DE FR GB IT**

(71) Anmelder: **BASF AKTIENGESELLSCHAFT**
**D-67056 Ludwigshafen (DE)**

(72) Erfinder: **Sterzel, Dr. Hans-Josef**
**Wasgauring 3**
**D-67125 Dannstadt-Schauernheim (DE)**
Erfinder: **Vogel, Dr. Herbert**
**Wolfsgrubenweg 17**
**D-67069 Ludwigshafen (DE)**
Erfinder: **Exner, Dr. Herbert**
**Goethestrasse 1**
**D-67165 Waldsee (DE)**
Erfinder: **Kratz, Dr. Detlef**
**Lutherstrasse 20**
**D-69120 Heidelberg (DE)**
Erfinder: **Brudermüller, Dr. Martin**
**Weberstrasse 7**
**D-68165 Mannheim (DE)**

## (54) Verfahren zur Herstellung von Milchsäureestern.

(57) Verfahren zur Herstellung von Milchsäureestern durch Fermentation von Zuckern enthaltenden Gemischen, Überführung der der Fermentation resultierenden Milchsäure in ihre Salze und anschließender Veresterung, dadurch gekennzeichnet, daß man

a) zu der Fermentationsbrühe Erdalkalicarbonat oder -hydrogencarbonat zugibt, um die entstehende Milchsäure zumindest zu 50 Mol-% zu neutralisieren,

b) die resultierende Fermentationsbrühe unter Zugabe von $NH_3$ und $CO_2$ auf einen pH-Wert von 7 bis 13 einstellt und die entstehenden Niederschläge abtrennt,

c) die so erhaltene und gereinigte Ammoniumlactatlösung mit einem Alkohol verestert.

EP 0 658 628 A1

Die Erfindung betrifft ein Verfahren zur Herstellung von Milchsäureestern durch Fermentation von Zuckern enthaltenden Gemischen, Überführung der bei der Fermentation resultierenden Milchsäure in ihre Salze und anschließender Veresterung.

Milchsäureester sind wichtige Zwischenprodukte bei der Herstellung von Lactid (I).

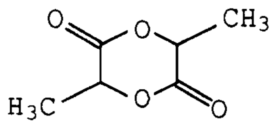

I

Das Lactid, speziell L- oder D-Lactid, hat eine große technische Bedeutung als Monomeres für die Herstellung von Polylactiden durch ringöffnende Polymerisation dieser Monomere.

Polylactide sind thermoplastisch verarbeitbar, weisen ein hohes mechanisches Eigenschaftsniveau sowie gute Sperreigenschaften gegen Wasserdampf und Sauerstoff auf. Unter üblichen Kompostierungsbedingungen werden sie schnell zu Milchsäure hydrolysiert und weiter bakteriell zu Wasser und Kohlendioxid abgebaut. Deshalb weisen Polylactide ein wachsendes Potential als abbaubares Verpackungsmaterial auf.

Optisch reine Lactide werden aus Gärungsmilchsäure hergestellt. Dabei weisen die Syntheseverfahren nach dem Stand der Technik einige gravierende Nachteile auf:

Nach dem Stand der Technik werden verdünnte Lösungen fermentierbarer Zucker durch Milchsäurebakterien zu L- oder D-Milchsäure, je nach Bakterienart, fermentiert. Da freie Milchsäure in Konzentrationen größer als 1 Gew.-% die Fermentation hindert, muß die Milchsäure neutralisiert werden, wozu üblicherweise vor allem Calcium- und/oder Magnesiumcarbonat verwendet wird. Bei genügend hoher Konzentration können die Ca- und/oder Mg-Lactate nach Abtrennen der Zellmasse durch Abkühlen der Gärungsbrühe auskristallisiert und damit teilweise aus der Gärungsbrühe abgetrennt werden. Die freie Milchsäure erhält man durch Umsetzung der Lactate mit konzentrierter Schwefelsäure. Damit werden jedoch etwa genausoviel zu deponierende Sulfate erzeugt wie Milchsäure, was die Abfallbilanz der kompostierbaren Polylactide in nachteiliger Weise belastet.

Es ist auch ein Verfahren beschrieben worden, welches diesen Nachteil nicht aufweist (WO 91/11527). Nach diesem Verfahren wird die Gärungsbrühe mit Ammoniak neutralisiert. Danach wird die Zellmasse von der Gärungsbrühe abgetrennt. Die Ammoniumlactatlösung wird mit einem Alkohol versetzt und bei Temperaturen von 160 °C bis 180 °C Kohlendioxid unter einem Druck von 150 bis 170 bar aufgepreßt.

Dabei wird unter Ammoniumhydrogencarbonat-Bildung der entsprechende Milchsäureester gebildet und abdestilliert. Dieses Verfahren weist jedoch andere gravierende Nachteile auf: Bei der Neutralisation der Gärungsbrühe mit Ammoniak werden nur maximale Milchsäurekonzentrationen in der Gärungsbrühe von 8 - 10 Gew.-% erreicht. Damit ist das Ammoniak-Verfahren von vornherein mit niedrigeren Raum-Zeit-Ausbeuten belastet. Zudem werden teure Hochdruckapparaturen benötigt. Das Gemisch aus Wasser, Milchsäureester und Alkohol muß hydrolysefrei voneinander getrennt werden, um eine Rückreaktion über die Hydrolyse des Milchsäureesters zu vermeiden.

Gemäß einer weiteren veröffentlichten Patentanmeldung (WO 93/00440) wird die Herstellung von Milchsäureestern oder Milchsäure aus einer Fermentationsbrühe beschrieben. Dort wird die gebildete Milchsäure mit Ammoniak neutralisiert und nach Abtrennung der Biomasse die Ammoniumlactat-Lösung durch Verdampfen von Wasser aufkonzentriert. Die aufkonzentrierte Ammoniumlactat-Lösung wird mit Schwefelsäure versetzt und damit die Milchsäure freigesetzt und Ammoniumsulfat teilweise auskristallisiert. Die Milchsäure wird mit einem $C_4$-$C_5$-Alkohol unter Abtrennen von Wasser verestert und der Ester dann weiter gereinigt und verarbeitet.

Somit hat auch dieses Verfahren den Nachteil, in der Fermentation mit den geringeren Raum-Zeit-Ausbeuten bei der Neutralisation der Milchsäure mit Ammoniak auskommen zu müssen. Außerdem wird in großen Mengen Ammoniumsulfat als Abfallstoff produziert.

Es bestand daher die Aufgabe, ein Verfahren zur Herstellung von Milchsäureestern zu finden, das die aufgeführten Nachteile nicht aufweist und insbesondere unter hoher Raum-Zeit-Ausbeute mit geringem apparativem und verfahrenstechnischem Aufwand abläuft.

Diese Aufgabe wird gelöst mit dem eingangs genannten Verfahren, das dadurch gekennzeichnet ist, daß man

a) zu der Fermentationsbrühe Erdalkalicarbonat oder -hydrogencarbonat zugibt, um die entstehende Milchsäure zumindest zu 90 Mol-% zu neutralisieren,

b) die resultierende Fermentationsbrühe unter Zugabe von $NH_3$ und $CO_2$ auf einen pH-Wert von 7 bis 13 einstellt und die entstehenden Niederschläge abtrennt,

c) die so erhaltene und gereinigte Ammoniumlactatlösung mit einem Alkohol verestert.

Eine bevorzugte Ausführungsform des Verfahrens besteht darin, daß man vor der Veresterung gemäß Verfahrensschritt c) die Ammoniumlactatlösung mit einem tertiären Alkylamin versetzt zur Bildung einer Trialkyl-Ammoniumlactatlösung, die dann ihrerseits mit dem Alkohol verestert wird.

Dabei wird das Ammoniumlactat bevorzugt mit mindestens äquimolaren Mengen eines tertiären Amins versetzt.

Eine bevorzugte Ausführungsform dieses Verfahrens besteht wiederum darin, daß man die Alkyl-Ammoniumlactatlösung vor der Veresterung aufkonzentriert, wobei es sich als vorteilhaft erweist, das bei der Aufkonzentrierung entweichende $NH_3$ und $CO_2$ in den Verfahrensschritt b) zurückzuführen. Besonders vorteilhaft ist es, das Verfahren kontinuierlich zu führen. Ein besonderer Vorteil des erfindungsgemäßen Verfahrens liegt darin, daß die Verfahrensschritte a) und b) im wesentlichen ohne eine Änderung der Temperatur des Reaktionsgemisches durchgeführt werden können. Schließlich kann das bei der Veresterung wieder frei werdende tertiäre Amin in das Verfahren rückgeführt werden.

Die in Verfahrensschritt b) stattfindende Umsetzung des Calciumlactats mit dem $CO_2$ und $NH_3$ kann wie folgt dargestellt werden:

$$Ca(Lac)_2 + CO_2 + 2NH_3 + H_2O \rightarrow 2NH_4Lac + CaCO_3$$

In der Figur ist ein Verfahrensschema für eine bevorzugte Ausführung des erfindungsgemäßen Verfahrens dargestellt.

Der erste Schritt besteht in der Fermentation einer Lösung von Melasse, Molke, Zucker oder Mischungen davon mit Zuckergehalten von 5 bis 15 % im Fermenter (1). Hierzu werden in an sich bekannter Weise (siehe z.B. WO 91/11527) Bakterien der Gattung Lactobacillus, die Milchsäure entweder in der D- oder der L-Form in hoher optischer Reinheit bilden, verwendet.

Dem Fermentationsansatz können als Nährstoffe eine komplexe Stickstoff-Quelle, Vitamine und Salze (vorzugsweise Phosphate) für die Bakterien zugemischt werden. Der Fermentationsreaktor (1) weist idealerweise die Verweilzeitcharakteristik eines Strömungsrohrs auf. Die Fermentation verläuft insbesondere bei Temperaturen von 37 bis 60 °C.

Die entstehende Milchsäure wird in Verfahrensschritt a) pH-kontrolliert durch Zupumpen einer wäßrigen Calciumcarbonat- und/oder Magnesiumcarbonat-Maische oder einer Maische aus den Hydrogencarbonaten zumindest zu 90 Mol-%, bevorzugt 95 Mol-% neutralisiert. Der pH-Wert wird bei der Fermentation bei vorzugsweise 4 bis 6, insbesondere bei 5,5 konstant gehalten. Nach einer ausreichenden Verweilzeit von 2 bis 20 h wird die Gärungsbrühe aus dem Fermenter abgezogen und ohne Zu- oder Abfuhr von Wärme der ersten Umsalzungsstufe (2), d.h. dem Verfahrensschritt b) zugeführt.

Dort wird die Gärungsbrühe mit dem im Fermenter entwickelten Kohlendioxid begast und gleichzeitig mit dem aus einer späteren Verfahrensstufe entstehenden Ammoniak versetzt. Der Ammoniakstrom wird derart dosiert, daß in (2) ein pH-Wert von 7 bis 13, insbesondere von 8,0 bis 9,5 eingestellt wird. Damit wird in Gegenwart der Zellmasse und störender Begleitstoffe Calcium- oder Magnesiumcarbonat gefällt und das Erdalkalilactat in Ammoniumlactat überführt. Damit bleibt die hohe Raum-Zeit-Ausbeute der Neutralisation mit zweiwertiger Base erhalten, und es wird ohne Verluste die äquivalente Menge Ammoniumlactat erhalten.

Hierbei werden die sonst störenden Stoffwechselprodukte an dem auskristallisierenden Calcium- oder Magnesiumcarbonat adsorbiert und größtenteils mitgefällt. Außerdem verbessert das Calcium- oder Magnesiumcarbonat überraschenderweise die Abtrennung der Feststoffmischung, z.B. in einem Filter oder einer Zentrifuge (3), erheblich. Verfahrensschritt b) kann in einem Rührkesselkaskade oder Strömungsrohr ausgeführt werden.

Man führt bevorzugt soviel Kohlendioxid, z. B. durch Kreislaufbegasung bei Drucken von 1 bis 3 bar zur daß der Gehalt an freiem $Ca^{2+}$ oder $Mg^{2+}$ $5 \cdot 10^{-4}$ Mol/Lit. nicht übersteigt, so daß alles Lactat umgesetzt ist. Eventuell vorhandenes überschüssiges Ammonium(hydrogen)carbonat wird in der Aufkonzentrierungsstufe zu Kohlendioxid und Ammoniak zersetzt, die in die erste Umsalzung (2) zurückgeführt werden.

Die Reaktionsmischung bleibt in allen Stadien niedrigviskos. Auch die gemeinsame Abtrennung der Feststoffe (3) erfolgt ohne Temperaturänderung und damit ohne zusätzlichen Aufwand thermischer Energie. Ein geringer Teil der Maische aus Zellmasse, Ca- oder Mg-Carbonat und Stoffwechselprodukten von 1 bis 20 % wird über die Filtrationsvorrichtung (4) ausgeschleust und weiter entwässert. Diese Masse kann als Silagestarter in der Landwirtschaft verwendet werden, oder sie wird verbrannt.

Die beim Entwässern gewonnene Mutterlauge enthält noch Ammoniumlactat und wird dem Filtrat zugegeben.

Die so gewonnene Ammoniumlactatlösung kann der zweiten Umsalzung, Stufe (5) zugeführt werden, wo sie mit einem tertiären Alkylamin vermischt wird. Der molare Anteil von tertiärem Amin zu $NH_4^+$ beträgt

bevorzugt 1,0 bis 1,2 : 1. Die Zumischung erfolgt ohne zusätzliches Abkühlen oder Aufheizen, also thermisch neutral, beispielsweise in einem Rührkessel oder Strömungsrohr. Als tertiäre Amine der Formel $NR_1R_2R_3$ (R = $C_1$-$C_6$-Alkyl) werden bevorzugt solche eingesetzt, deren Siedepunkte über dem des Wassers liegen, beispielsweise Tripropylamin oder Tributylamin. In der darauffolgenden Stufe (6) kann die Lösung durch Zuführung thermischer Energie, vorzugsweise mehrstufig, unter Abdestillieren von Wasser, Ammoniak und überschüssigem Kohlendioxid aus Stufe (2) aufkonzentriert werden. Wasser wird von Kohlendioxid und Ammoniak in Kolonne (7) getrennt und Kohlendioxid und Ammoniak, die noch geringe Mengen Wasser enthalten können, Stufe (2) zugeführt. Zu beachten ist, daß kein organisches tertiäres Amin in die Stufe (2) gelangt, denn es könnte über die zurückgeführte feuchte Zellmasse die Fermentation stören.

Die gereinigte Ammoniumlactatlösung oder die, ggf. aufkonzentrierte, Lösung mit $NR_3H^+Lac^-$ als Wertstoff wird der Veresterungsstufe (8), d.h. dem Verfahrensschritt c) zugeführt und dort mit einem einwertigen Alkohol, insbesondere einem Alkohol mit 2 - 10 C-Atomen, z.B. Butanol-1, Pentanol-1 oder Hexanol-1, oder einem höhermolekularem Alkohol, z.B. einem langkettigen Mono- oder Diol mit mehr als 10 C-Atomen, verzweigten Diolen oder Polyolen wie längerkettigen Homologen des Pentaerythrits oder insbesondere Polyethylenoxiden mit Molmassen von 180 bis 1000 g/Mol, wie z.B. $HO-(CH_2-CH_2-O)_n-CH_2-CH_2-OH$ mit n = 2 - 25, bevorzugt in Abwesenheit eines Katalysators bei Temperaturen von 70 °C bis 200 °C unter Normaldruck oder im Vakuum (1-1000 mbar, bevorzugt 15-200 mbar), verestert. Im Falle des Einsatzes der $NR_3H^+Lac^-$-Lösung wird dabei das tertiäre Amin, vermischt mit Wasser, abdestilliert und zu Stufe (5) zurückgeführt. Die Veresterung wird bevorzugt mittels einer Rührkesselkaskade mit gemeinsamem Brüdenabzug durchgeführt. Zur Veresterung kann ein Molverhältnis von Alkohol zu Milchsäure z.B. von 0,5 bis 5 : 1 eingesetzt werden.

Der Austausch von $NH_4^+$ gegen $NR_3H^+$ kann insbesondere durchgeführt werden, um bei der Veresterung die Bildung von Milchsäureamid als Nebenreaktion und damit entsprechende Ausbeuteverluste zu vermeiden. Die Mischung aus Milchsäureester und überschüssigem Alkohol kann destillativ aufgetrennt werden.

Die erfindungsgemäßen Milchsäureester können, gegebenenfalls nach einer Umesterung, z.B. mit einem höheren Alkohol, zum Lactid umgesetzt werden. Die an sich bekannte Lactidbildung stellt eine innere Umesterung dar, wobei der zunächst zur Veresterung eingesetzte Alkohol wieder freigesetzt wird und erneut in das Verfahren rückgeführt werden kann.

Das erfindungsgemäße Verfahren ist aus verschiedenen Gründen verfahrenstechnisch günstig und vorteilhaft:

Auf den Energieaufwand zur Abkühlung der Gärungsbrühe zum Auskristallisieren des Calciumlactat kann verzichtet werden, ebenso auf die Investitionen von Kristallisator und Kühlaggregat.

Unter Verwendung einer reinen Zuckerlösung erhält man nach dem Verdampfer (6) eine konzentrierte Ammoniumlactat-Lösung, die bezogen auf das gelöste Ammoniumlactat weniger als 1 Gew.-% Nebenprodukte enthält.

Die Fermentation verläuft unter hoher Raum-Zeit-Ausbeute mit den daraus folgenden Vorteilen geringer Investitions- und Energiekosten; bei der Neutralisation in Stufe a) werden Milchsäurekonzentrationen von 13 bis 15 Gew.-% erreicht. Rohstoffe, Nährstoffe und Stoffwechselprodukte können weitgehend in das Verfahren zurückgeführt werden, damit wird ebenfalls die Ausbeute erhöht; es werden keine Hochdruckapparate benötigt, es treten keine Ströme mit hohen Viskositäten auf; die Anzahl der Apparate ist niedrig.

Aufgrund der milden Reaktionsbedingungen können hohe optische Reinheiten erreicht werden.

## Beispiele

Die im folgenden aufgeführten Beispiele wurden bis auf die Fermentation diskontinuierlich durchgeführt.

### Fermentation (Verfahrensstufe a)

80 g trockene Bierhefe werden mit 5 l Wasser in einem 15 l Fermenter sterilisiert. Hierzu wird nach dem Abkühlen auf 50 °C eine zuvor separat sterilisierte Lösung aus 120 g Glucose, 10 ml konzentrierter Phosphorsäure und 0,3 g Tween 80 (Fa. ICI) in 5 l Trinkwasser zugegeben. Die Kultur wird mit 100 ml einer maximal 12 h alten Kultur von Lactobacillus casei ATCC 27139, welche in MRS-Medium (3. Appl. Bacteniol. 23, 130 (1960)) angezogen wurde, beimpft.

Die Fermentationsbrühe wird bei 50 °C mit 250 upm unter Zugabe von $CaCO_3$ gerührt. Nach etwa 20-24 h wird die Kultur von batch-Verfahrensweise auf kontinuierliche Betriebsweise umgestellt.

Zu diesem Zeitpunkt beträgt die Ca-Lactat Konzentration 85-110 g/l. Die Umstellung auf kontinuierlichen Betrieb erfolgt durch Zuführung von frischem Medium mit der oben beschriebenen Komposition. Der Zufluß von frischem Medium und von zur Aufrechterhaltung eines konstanten pH-Wertes nötigen Lauge ($CaCO_3$-Suspension) wird so eingestellt, daß die Verweilzeit 15,5 h beträgt. Die Produktkonzentration im Ablauf des Fermentes beträgt ca. 12 Gew.-% bezogen auf Milchsäure.

Erste Umsalzung (Verfahrensstufe b)

18,9 kg einer wie oben beschriebenen Fermentationsbrühe mit einem Gehalt an Calciumlactat, der ca. 12 Gew.-% an Milchsäure entspricht (entsprechend ca. 25,2 Molen Milchsäure), mit einem Ausgangs-pH-Wert von 5,2 werden bei 50 °C gehalten.

Innerhalb von 40 min wurde die Brühe unter starkem Rühren mit 430 g Ammoniak sowie mit ca. 570 g Kohlendioxid versetzt. Zu Beginn der Reaktion stieg der pH-Wert auf 9,4, um während der Umsetzung auf 8,2 abzusinken. Weiteres Einleiten von $CO_2$ brachte keine weitere Absenkung des pH-Werts. Während der Umsetzung hellte sich die braune Brühe aufgrund der Bildung des Calciumcarbonats nach beige auf. Die Reaktionsmischung bleibt niedrigviskos. Nach Abstellen des Rührers setzte sich der Feststoff wesentlich schneller ab als die Zellmasse der reinen Fermentationsbrühe.

Die Feststoffmischung ließ sich durch Filtration aus der Reaktionsmischung bei ebenfalls ca. 50 °C schnell und problemlos abfiltrieren. Nach Waschen mit wenig Wasser werden 2,76 kg feuchter Filterkuchen erhalten. Die Masse des getrockneten Filterkuchens beträgt 1,40 kg, der Anteil an Calciumcarbonat 1,25 kg.

Die so erhaltene Ammoniumlactatlösung kann unmittelbar zur Veresterung eingesetzt werden, sie kann aber auch zunächst noch einer weiteren Umsalzung unterzogen werden.

Zweite Umsalzung (fakultative Maßnahme)

1000 g einer 15 %igen Lösung von Ammoniumlactat werden stöchiometrisch mit Triethylamin vermischt (200 ml = 1.5 mol) und unter Rückfluß 3 h erhitzt. Das entweichende Ammoniak wird in einer Waschflasche in Wasser absorbiert.

Die so erhaltene Triethylammoniumlactat-Lösung wies ein Gehalt von 26.3 Gew.-% $Et_3NH$-Lac auf. Die Lösung war sogleich für Veresterungen einsetzbar.

Veresterung (Verfahrensstufe c)

a) 500 g $NH_4$-Lactat-Lösung wurden im Vakuum (100 mbar, 43 °C) eingeengt. Es wurden ca. 270 g Wasser abdestilliert.
Die verbliebene Lösung enthält ca. 0,5 mol $NH_4$-Lactat. Dieses wurde mit 120 g Pentanol (ca. 1,5 mol) versetzt und unter Wasserauskreisung verestert, bis keine Wasserabscheidung mehr erkennbar war.
Das im Sumpf verbliebene Veresterungsgemisch wurde anschließend bei 100 mbar fraktioniert destilliert. Dabei wurden insgesamt 72.3 g Pentyllactat durch Destillation gewonnen. Dies entspricht einer Ausbeute vom 90.4 %.

b) 357 g einer 80 %igen Lösung von L-Ammoniumlactat (2,7 mol) und 200 g Triethylenglykol (1,33 mol) werden zusammengegeben. Unter Rühren wird bei 160 °C über eine Vigreux-Kolonne ein Wasser/Ammoniak Gemisch abdestilliert. Dann wird bei der gleichen Temperatur ein Vakuum von 20 mbar angelegt und weiter abdestilliert. Es werden insgesamt 95 g $H_2O$ erhalten. Umsatz: 80 %. Das Produkt enthält folgende Hauptkomponenten (GC-Flächen%) und kann zur Lactidherstellung verwendet werden:
Milchsäure 1 %, Lactid 6 %, Triethylenglykol 12 %, Triethylenglykolmonolactat 39 %, Triethylenglykoldilactat 28 %.

c) Es wird wie in Beispiel b) beschrieben vorgegangen. Einsatzstoffe: 179 g (1,35 mol) 80 %iges Ammoniumlactat, 276 g (0,67 mol) Lutrol E400 (Polyethylenglykol $C_{18}H_{38}O_{10}$).
Es werden 51 g Destillat erhalten. Umsatz: 85 %.
Das GC-analytisch untersuchte Gemisch enthält Milchsäure (2,4 %), Lactid (35 %), Lutrol E400 (29 %) und Lutrolmono- und dilactat. Das Rohgemisch kann zur Lactidherstellung verwendet werden.

d) In einem Rührkolben wurden ca. 300 ml Triethylammoniumlactat (TEAL) (95 %ige Lsg.), erhalten durch eine zweite Umsalzung (wie oben beschrieben), vorgelegt. Die Lösung wurde langsam erwärmt. Bei ca. 115 °C erfolgte die thermische Spaltung des Lactats. Triethylamin und Wasser konnte azeotrop abdestilliert werden. Die Innentemperatur wurde weiter bis auf ca. 180 °C erhöht und dann kontinuierlich n-Pentanol (240 ml(h) und TEAL (88 g/h, 95 %) zudosiert. Überschüssiger Alkohol und Pentyllactat wurden kontinuierlich über Kopf abgezogen. Das Destillat enthielt neben Triethylamin, Wasser, n-Pentanol und 25 Gew.-% n-Pentyllactat (= 95 % Ausbeute).

e) 747 g einer 80 %igen Lösung von L-Triethylammoniumlactat (3,5 mol) und 186 g Diethylenglykol (1,75 mol) werden zusammengegeben. Unter Rühren wird bei 120 °C im Vakuum (40 mbar) über eine Vigreux-Kolonne ein Gemisch aus Wasser und Triethylamin abdestilliert (360 g).

Anschließend wird die Temperatur auf 140 °C erhöht worauf weitere 112 g $NEt_3/H_2O$ abdestillieren. Umsatz: 95 %.

Das Produkt enthält folgende Hauptkomponenten (GC-Flächen%) und kann zur Lactidherstellung verwendet werden:

Diethylenglykol 2 %, Lactid 19 %, Diethylenglykolmonolactat 22 %, Diethylenglykoldilactat 57 %.

## Patentansprüche

**1.** Verfahren zur Herstellung von Milchsäureestern durch Fermentation von Zuckern enthaltenden Gemischen, Überführung der bei der Fermentation resultierenden Milchsäure in ihre Salze und anschließende Veresterung, dadurch gekennzeichnet, daß man

a) zu der Fermentationsbrühe Erdalkalicarbonat oder -hydrogencarbonat zugibt, um die entstehende Milchsäure zumindest zu 90 Mol-% zu neutralisieren,

b) die resultierende Fermentationsbrühe unter Zugabe von $NH_3$ und $CO_2$ auf einen pH-Wert von 7 bis 13 einstellt und die entstehenden Niederschläge abtrennt,

c) die so erhaltene und gereinigte Ammoniumlactatlösung mit einem Alkohol verestert.

**2.** Verfahren nach Anspruch 1, dadurch gekennzeichnet, daß man vor der Veresterung gemäß Verfahrensschritt c) die Ammoniumlactatlösung mit einem tertiären Alkylamin versetzt zur Bildung einer Alkyl-Ammoniumlactatlösung.

**3.** Verfahren nach Anspruch 2, dadurch gekennzeichnet, daß man die Alkyl-Ammoniumlactatlösung vor der Veresterung aufkonzentriert.

**4.** Verfahren nach Anspruch 3, dadurch gekennzeichnet, daß man das bei der Aufkonzentrierung abgetrennte $NH_3$ und $CO_2$ in den Verfahrensschritt b) zurückführt.

**5.** Verfahren nach Anspruch 1, dadurch gekennzeichnet, daß das Verfahren kontinuierlich geführt wird.

**6.** Verfahren nach Anspruch 2, dadurch gekennzeichnet, daß das Ammoniumlactat in Verfahrensschritt c) mit mindestens äquimolaren Mengen eines tertiären Amins versetzt wird.

**7.** Verfahren nach Anspruch 1, dadurch gekennzeichnet, daß die Verfahrensschritte a) und b) im wesentlichen ohne eine Änderung der Temperaturen des Reaktionsgemisches durchgeführt werden.

**8.** Verfahren nach Anspruch 2, dadurch gekennzeichnet, daß das bei der Veresterung in Verfahrensschritt c) frei werdende tertiäre Amin in das Verfahren rückgeführt wird.

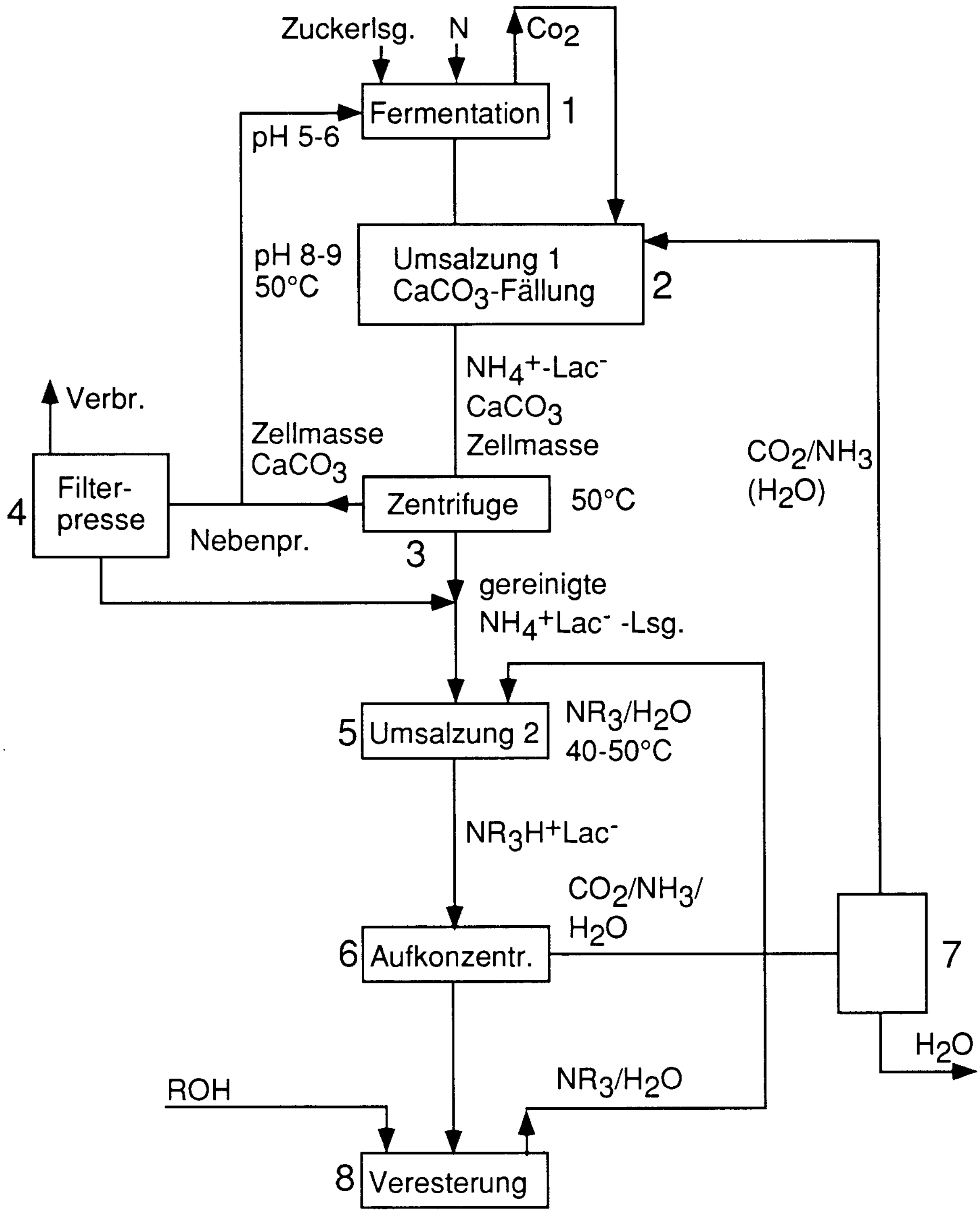
Zuckerlsg.
N
Co2
Fermentation
1
pH 5-6
pH 8-9
50°C
Umsalzung 1
CaCO3-Fällung
2
NH4+-Lac-
CaCO3
Zellmasse
Verbr.
Zellmasse
CaCO3
CO2/NH3
(H2O)
Filter-
presse
4
Zentrifuge
50°C
Nebenpr.
3
gereinigte
NH4+Lac- -Lsg.
5
Umsalzung 2
NR3/H2O
40-50°C
NR3H+Lac-
CO2/NH3/
H2O
6
Aufkonzentr.
7
H2O
ROH
NR3/H2O
8
Veresterung

Europäisches Patentamt

# EUROPÄISCHER RECHERCHENBERICHT

Nummer der Anmeldung
EP 94 11 8848

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | BE-A-676 731 (MINISTERSTWO PRZEMYLSLU SPOZYWCZEGO I SKUPU)<br>* Ansprüche 1-7 *<br>--- | 1,3,5,7 | C12P7/56<br>C07C59/08 |
| D,Y | WO-A-91 11527 (BATELLE MEMORIAL INSTITUTE)<br>* Anspruch 1 *<br>--- | 1,3,5,7 | |
| D,Y | WO-A-93 00440 (E. I. DU PONT DE NEMOURS AND COMPANY)<br>* Ansprüche; Abbildung 2 *<br>--- | 1,3,5,7 | |
| Y | US-A-3 125 494 (R. L. SNELL ET AL.)<br>* Spalte 2, Zeile 39 - Zeile 45; Anspruch 1 *<br>--- | 1 | |
| A | US-A-4 698 303 (R. B. BAILEY)<br>* Anspruch 1 *<br>----- | 2 | |
| | | | RECHERCHIERTE SACHGEBIETE (Int.Cl.6) |
| | | | C12P<br>C07C |

Der vorliegende Recherchenbericht wurde für alle Patentansprüche erstellt

| Recherchenort | Abschlußdatum der Recherche | Prüfer |
|---|---|---|
| DEN HAAG | 10.April 1995 | Coucke, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument